(19) Europäisches Patentamt European Patent Office Office européen des brevets

(11) **EP 1 408 882 B1**

(12) **EUROPEAN PATENT SPECIFICATION**

(45) Date of publication and mention
of the grant of the patent:
**11.02.2009 Bulletin 2009/07**

(51) Int Cl.:
**A61F 2/24** (2006.01)

(21) Application number: **02748256.1**

(22) Date of filing: **26.07.2002**

(86) International application number:
**PCT/US2002/023904**

(87) International publication number:
**WO 2003/009785 (06.02.2003 Gazette 2003/06)**

(54) **METHOD OF CUTTING MATERIAL FOR USE IN IMPLANTABLE MEDICAL DEVICE**

SCHNEIDEVERFAHREN FÜR MATERIALIEN ZUR VERWENDUNG IN MEDIZINISCHEN IMPLANTATEN

PROCEDE DE DECOUPE DE MATERIAU UTILISE DANS UN DISPOSITIF MEDICAL IMPLANTABLE

(84) Designated Contracting States:
**AT BE BG CH CY CZ DE DK EE ES FI FR GB GR IE IT LI LU MC NL PT SE SK TR**

(30) Priority: **26.07.2001 US 308268 P**

(43) Date of publication of application:
**21.04.2004 Bulletin 2004/17**

(73) Proprietor: **3F Therapeutics, Inc**
**Lake Forest, CA 92630 (US)**

(72) Inventor: **CALI, Douglas, S.**
**Mission Viejo, CA 92691 (US)**

(74) Representative: **Needle, Jacqueline**
**Beck Greener**
**Fulwood House,**
**12 Fulwood Place,**
**London WC1V 6HR (GB)**

(56) References cited:
**WO-A-01/54624**     **WO-A-99/30884**
**WO-A-02/053069**    **US-A- 6 129 758**
**US-B1- 6 254 636**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**Description**

This invention relates to a method and apparatus for creating an implantable medical device.

Medical devices are often surgically implanted into a patient in order to assist or replace diseased tissue. For instance, a prosthetic device such as an artificial heart valve can be implanted to replace a defective natural heart valve. It is important that such prosthetic devices are durable, as failure of the device may have drastic consequences for the patient. As can be appreciated, a prosthetic device that wears out prematurely may put a patient at substantial risk, both because of the possibility of early, sudden failure of the device and because of additional surgery that may be required to replace the device.

Some implantable medical devices comprise two or more members or segments of material that are assembled to form the device. The manner in which the segments of material are formed can significantly affect the durability of the device. For example, if the segments are formed by being cut out of a larger portion of material, the edges of the cut segments may be especially susceptible to premature wear. Also, imprecise cutting or inconsistencies between cut segments may negatively affect both the operability and durability of the assembled prosthetic device.

US-A-6,129,758 describes a methodology and surgical instruments for cutting a precisely sized and shaped geometric pattern with which an atrioventricular valve reconstruction can be accomplished.

The present invention seeks to provide an implantable medical device where the cut edges resist wear.

According to a first aspect of the present invention there is provided a method of creating an implantable medical prosthesis, comprising:

providing a sheet of flexible synthetic or explanted biological source material having at least two tissue layers; and cutting a segment of tissue out of the sheet of source material with a laser beam;
said cutting comprising operating a laser at a power and pulse rate such that said beam welds the layers of the source material together along a laser cut edge without significantly burning the source material adjacent the cut edge:

wherein the laser is operated in a pulsed manner, supplying between about 0.005-0.5 joules of laser energy per pulse, with a laser spot size of about 0.05-0.13mm (0.002-0.005 inches) in diameter.

The invention also extends to apparatus for creating an implantable medical prosthesis, said apparatus comprising:

a laser system for creating a focussed laser beam for cutting a segment of tissue out of a sheet of a flexible source material having at least two tissue layers;
the laser system operating a laser at a power and pulse rate such that the focussed laser beam welds the layers of the source material together along a laser cut edge without significantly burning the source material adjacent the cut edge;
wherein the laser is operated in a pulsed manner, supplying between about 0.005-0.5 joules of laser energy per pulse, with a laser spot size of about 0.05-0.13mm (0.002-0.005 inches) in diameter.

Embodiments of the present invention will hereinafter be described, by way of example, with reference to the accompanying drawings, in which:

Figure 1 is a perspective view of a prosthetic aortic heart valve constructed by joining three independently formed leaflets together,

Figure 2 shows a flat pattern for a leaflet to be used in constructing the heart valve of Figure 1,

Figure 3 shows two adjacent leaflets of the valve of Figure 1 during valve assembly,

Figure 4 is a top view showing the leaflets of Figure 3 folded over each other to form a commissural tab,

Figure 5 is a scanning electron microscope image of an edge of an equine pericardium segment that has been cut with a razor,

Figure 6 is a schematic view of a plotted laser cutting apparatus for precision cutting of segments for implantable medical devices,

Figure 7 is a plan view showing several aortic valve leaflets arranged to be cut by the plotted laser apparatus of Figure 6,

Figure 8 is a scanning electron microscope image of an edge of a segment of equine pericardium that has been cut with the plotted laser cutting apparatus of Figure 6, and

Figure 9 is a schematic view of another embodiment of a plotted laser cutting apparatus for precision cutting of segments for implantable medical devices.

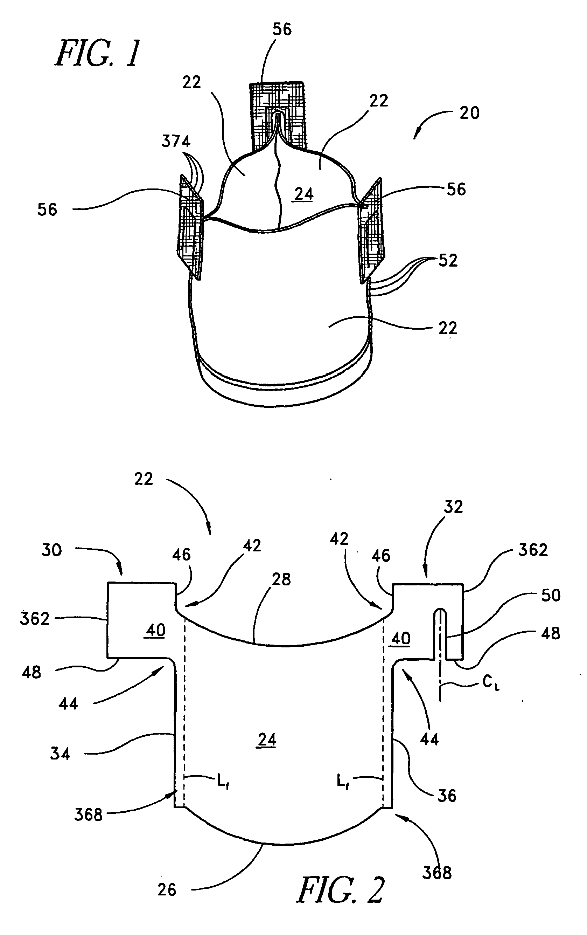

In methods of the invention segments are cut out and used to construct an implantable medical prosthesis. One type of prosthesis that particularly benefits from use of the present invention is a replacement heart valve having one or more leaflets that are cut from a source material and assembled to form the valve. Figures 1-4 illustrate a prosthetic aortic heart valve 20 constructed in accordance with an embodiment of the invention. However, it will be appreciated that the invention is not limited to the construction of heart valves and that other types of implantable medical prostheses may be constructed as described herein.

the aortic heart valve 20 of Figures 1-4 comprises three leaflets 22 that are cut out of a generally flat, flexible source material. Each of the three leaflets 22 is cut out according to the pattern shown in Figure 2. As shown, each leaflet 22 has a main body 24 that is scalloped at both its proximal and distal ends 26, 28. First and second distal tab portions 30, 32 extend outwardly from corresponding first and second side edges 34, 36 of each leaflet's main body 24. The tabs 30, 32 are substantially rectangular in shape and extend distally beyond the distal end 28 of the main body 24.

Each of the tabs 30, 32 communicates with the leaflet main body 24 through a neck portion 40. Curved transition edges 42, 44 connect an inner edge 46 of each tab 30, 32 with the distal end 28 of the leaflet 22, and a proximal edge 48 of each tab 30, 32 with the corresponding side edge 34, 36 of the leaflet 22. An elongate slot 50 is formed in the second tab 32. The slot 50 extends distally from the proximal edge 48 of the tab to a point just distal of the distal-most edge 28 of the leaflet main body 24.

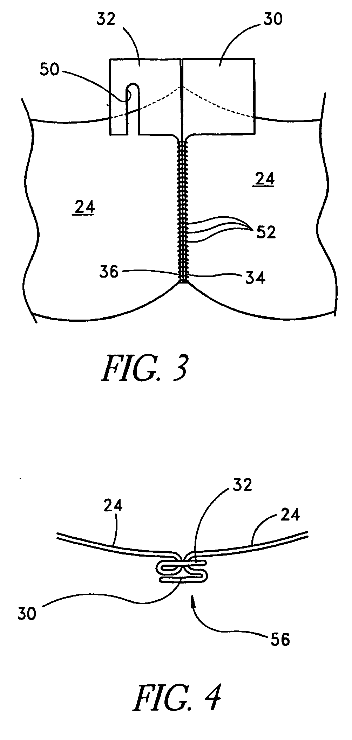

With reference next to Figure 3, adjacent leaflets are connected by aligning the first outer edge 34 of one leaflet with the second outer edge 36 of the adjacent leaflet so that the inner faces of the leaflets engage one another. The side edges 34, 36 are sutured together using a series of locked stitches 52 arranged along a fold line $L_F$ adjacent each side edge 34, 36.

The series of sutures 52 terminates prior to reaching the proximal edge 48 of the tabs 30, 32, with the last suture being placed proximal of the proximal transition edge 44. The tabs 30, 32 are then folded backwardly along the fold line $L_F$ so as to overlap the outer surface of their respective leaflets 22, as shown in Figure 3. With reference next to Figure 4, the adjacent first and second tabs 30, 32 are folded over one another in order to form commissural tabs 56. More specifically, the second tab 32 is folded so that the slot 50 straddles the neck portions 40 of both tabs 30, 32. The first tab 30 is folded opposite the second tab 32 and generally aligned with the second tab 32, as shown in Figure 4. The folded tabs 30, 32 are then sewn together in order to form the commissural tabs 56 shown in Figure 1.

In the illustrated embodiment, each of the leaflets 22 is substantially identical in shape. It is to be understood, however, that other prosthetic devices may employ segments of varying sizes and shapes. For example, a prosthetic mitral heart valve can employ two leaflets which are shaped differently from one another. However to maintain consistency in manufacture, the respective leaflets preferably are substantially identical in size and shape from valve to valve. Additionally, prosthetic devices such as surgical patches may desirably be produced in several sizes and shapes.

Replacement valves such as the aortic valve 20 illustrated in Figures 1-4 are used to replace diseased natural valves. The natural valve is cut out of its place and removed, leaving a valve annulus and a plurality of downstream attachment locations. The inflow annulus of the replacement valve is configured to fit into the valve annulus vacated by the native aortic valve. The commissural attachment tabs 56 can be attached to the aorta at points vacated by the native valve's commissural attachment locations. The replacement valve thus totally replaces the native valve.

Once installed, the replacement valve functions much the same as a native aortic valve. During systole, the leaflets 22 are forced apart so that blood flows freely through the valve 20 and into the aorta. During diastole, the leaflets are drawn toward each other and approximate each other, thus sealing the valve. The commissural attachment tabs 56 help prevent the valve leaflets from prolapsing during diastole.

In the illustrated embodiment, the leaflets can be constructed of biological or synthetic materials. For example, explanted human or animal tissue, such as bovine, porcine and kangaroo pericardium tissue may be appropriately used. Synthetic material, such as polyesters, Teflon®, fluoropolymers, woven or knitted cloth, etc. can also be used. Of course, biological and synthetic materials not listed above can be used if appropriate. Leaflet materials for the illustrated heart valve can be selected using a general guideline that the more pliable, thin and strong a material is, the better. Additionally, it is advantageous for the material to be as nonthrombogenic as possible.

In a preferred embodiment, the flexible material comprises equine pericardium that has been crosslinked and fixed in a low-concentration, buffered glutaraldehyde solution. Leaflets formed from this material are pliable and easy to open and close.

Equine pericardium that has been treated as discussed above can be supplied as a generally flat, thin and flexible sheet of material from which a plurality of leaflets can be cut. Other source materials, such as bovine pericardium and woven cloth, can also be obtained in flat sheets. Still further source materials may be obtained in irregular or curved shapes. For example, segments of intestinal tissue, some knitted cloths and some extruded polymers can be supplied having generally tubular geometry. Segments can be cut from such suitable source materials and then assembled to form the desired prosthesis. Various cutting media and methods, such as a razor, die cutter, laser or jet of fluid and/or particles can be used to cut segments from source material. In a preferred embodiment of the aortic heart valve discussed above, individual valve leaflets are cut from a sheet of treated equine pericardium.

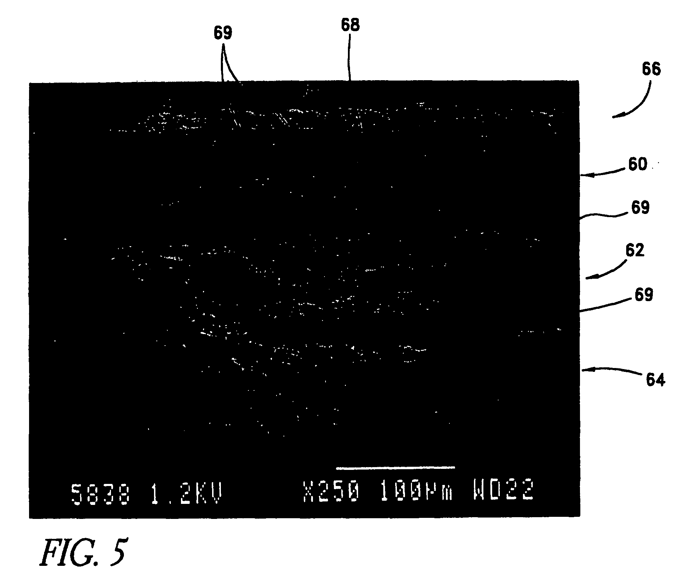

With next reference to Figure 5, equine pericardium has a laminar structure with three fibrous layers, the visceral 60, serosa 62, and parietal layers 64. Applicant has discovered that cutting equine pericardium using a contact-type cutter such as a razor or cutting die has a tendency to delaminate one or more of the layers along the cut edges. Figure 5 is a scanning electron microscope image of an equine pericardium segment edge 66 that has been cut with a razor.

As can be seen in Figure 5, each of the layers 60, 62, 64 has a generally different consistency. Additionally, the fibrous material 68 within each layer has several discontinuities and gaps 70. In this configuration, the cut edge 66 is especially susceptible to degradation due to external factors. For example, a fluid such as blood can fill some of the gaps 69 between the layers 60, 62, 64 or fibers 68 and can act as a wedge gradually disconnecting the layers or fibers from one another. Over time such delaminations would advance beyond just the cut edge, and may compromise the performance and strength of the prosthetic segment.

Delaminations of the fibrous layers of a heart valve leaflet can disrupt valve operation and significantly impair valve durability. For example, blood that enters between delaminated layers can cause a cuspal hemotoma or lead to calcification of the valve due to increased turbulence. Additionally, the strength of the leaflet can be reduced. Accordingly, it is desirable to reduce or eliminate delamination of the pericardium layers when constructing valves.

Other flexible materials used for heart valves, especially pericardial tissues, may have similar laminar structure, and may be subject to similar issues with regard to delamination. Challenges also arise when cutting synthetic materials such as woven or knit polymers, because the cut filaments or yarns may have a tendency to fray. Such fraying can cause problems similar to delamination.

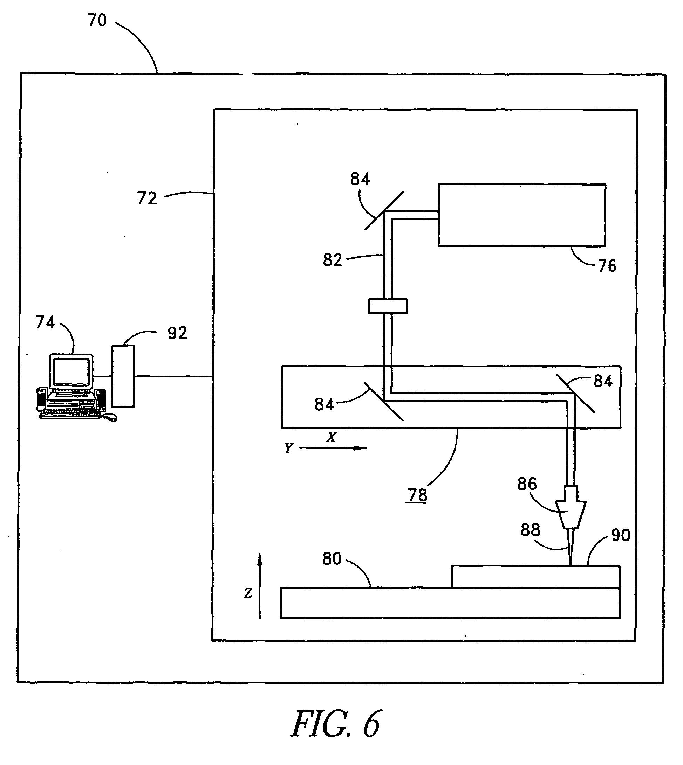

In accordance with one embodiment, a laser cutting apparatus 70 is provided for cutting prosthetic segments from source material 90. With reference specifically to Figure 6, the laser cutting apparatus 70 comprises a laser system 72 and a computer 74. The laser system 72 comprises a laser tube assembly 76, a motion system 78 and a support platform 80. The laser tube assembly 76 is configured to create a laser beam 82 which is directed through a series of optic elements such as mirrors 84 and lenses 86 in order to direct a focused laser beam 88 on the support platform 80, which is configured to support the source material 90. The focused laser beam 88 is configured to cut through the source material 90 in order to cut out a segment according to a prescribed pattern.

The motion system 78 preferably is arranged to selectively locate and move the position of the focused laser beam 88 relative to the platform 80 in order to cut the segment out of the source material 90. In the illustrated embodiment, the motion system 78 can move the laser beam's position along horizontal X and Y axes. The support platform 80 is vertically movable along a vertical Z axis. It is to be understood that, in other embodiments, other types of motion systems can be employed.

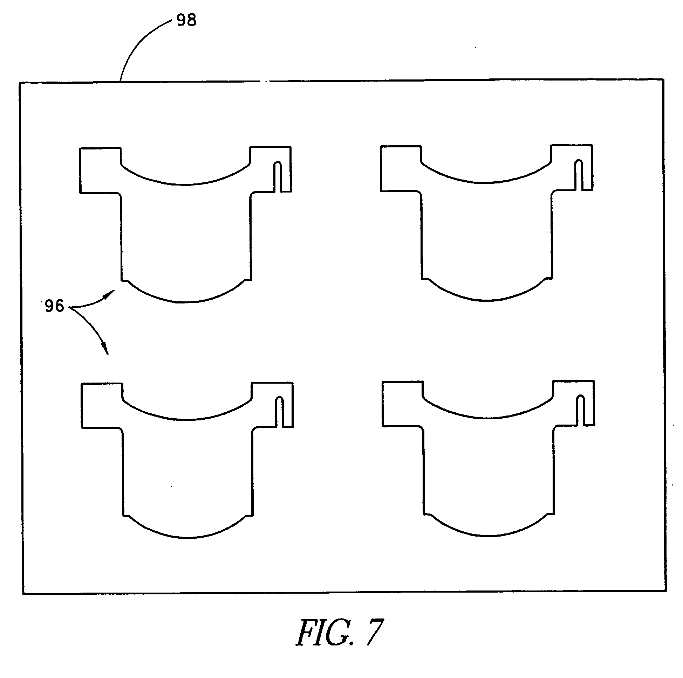

The computer 74 preferably controls the laser system 72 via a printer driver 92, which communicates data from the computer 74 to the laser system 72 in order to control laser parameters and motion. In the illustrated embodiment, a computer assisted design (CAD) software program, such as Corel Draw®, is hosted by the computer 74. The CAD software is used to create designs of segments that will be cut. Figure 7 shows a cutting pattern or template 96 created by CAD software. The template 96 functions as a target for the laser. The illustrated template 96 is configured so that four valve leaflets will be cut from a sheet 98 of source material.

In a preferred embodiment, the CAD software also functions as a command interface for submitting cutting patterns 96 to the laser system 72 through the printer driver 92. When directed to do so by the computer 74 and printer driver 92, the laser system 72 precisely cuts the patterns 96 from the source material 90.

The laser cutting apparatus 70 is configured to have a pulse power, cutting speed, and number of pulses per inch that will impart sufficient energy to vaporize portions of the source material along a cut line in order to cut the desired segment shape, and to at least partially melt the cut edges. Melting the cut edges effectively fuses or welds the layers and fibrous matter together.

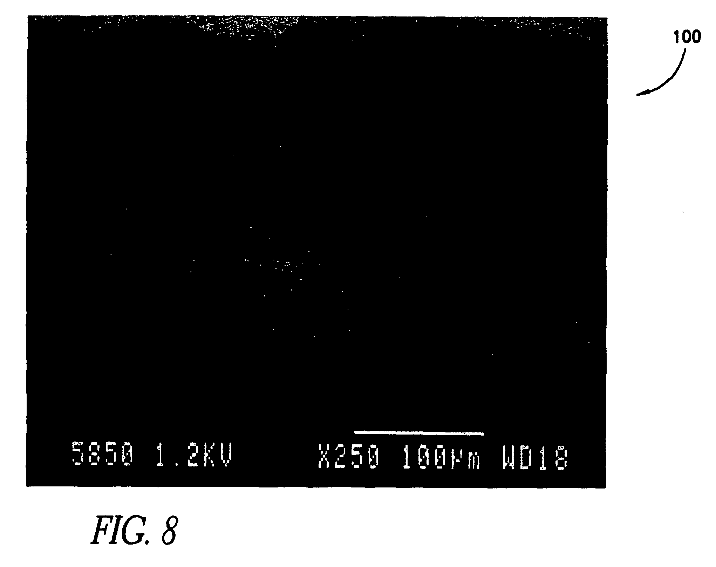

Welding of the edges is especially advantageous for laminar materials such as pericardium, because the melted edge resists delamination. Figure 8 is a scanning electron microscope image taken along a cut edge 100 of a sample of equine pericardial tissue that has been cut using a laser. When compared with Figure 5, Figure 8 shows that the characteristics of the laser-cut edge 100 are much different than the a razor-cut edge 66. As shown in Figure 8, the visceral, serosa and parietal layers are no longer distinguishable when the material has been laser cut. Additionally, the generally fibrous, layered character of the pericardium has been changed along the cut edge 100. Applicant has found that heart valve leaflets with melted edges exhibit dramatically increased durability over leaflets that have been cut using more traditional die-cutting or razor-cutting methods.

An issue that arises during laser cutting is management of thermal energy. Excessive thermal energy absorbed by a source material such as pericardium can bum the material. Burning of the material can result in several types of damage. For example, the burned material can become stiff and brittle or can become biased to bend in a particular direction. Further characteristics of burning include discoloration or even charring of the material.

Burned portions of a segment of material can jeopardize the integrity and durability of the entire segment, and of a prosthesis constructed using that segment. For example, a stiffened or biased portion of a prosthetic heart valve leaflet will not move in the same manner as the rest of the leaflet during opening and closure of the valve. The hemodynamic performance of the valve thus could be compromised. Further, damage caused by burning of the material generally weakens the material and could reduce the durability of the valve. As such, it is desirable to weld the material at the cut edge, but avoid communicating thermal energy into the cut segment beyond the edge.

Excessive burning of the laser cut edge can also have a negative impact. If excessive laser energy is applied to the cut edge, it is more likely that thermal energy will be conducted beyond the edge and into the segment, resulting in tissue necrosis. Additionally, the tissue at an excessively-burned edge may have a somewhat inconsistent thickness, having portions that are significantly thicker than other portions or developing beads of melted material. Discoloration of the cut edge can indicate application of excessive thermal energy. Inconsistencies in the edge make the segment more difficult to work with during manufacture and can affect performance of the segment. As such, it is desirable to weld the material at the cut edge in a manner so that the melted edge is relatively uniform in thickness and consistency and exhibits minimal, if any, beading.

In a preferred embodiment, a $CO_2$ laser is used to laser cut heart valve leaflets out of a sheet of equine pericardial tissue about .35-.55 mm thick The laser system preferably is an M-series laser engraving and cutting system available from Universal Laser Systems, Inc. This device employs a 30 watt, pulsed, sealed $CO_2$ laser. The $CO_2$ laser produces laser light with a characteristic wavelength of 10.6 $\mu$m. Most non-metals, including equine pericardial tissue, are highly absorptive of laser energy at this wavelength, and also exhibit low thermal conductivity to such laser energy. Hence, the $CO_2$ laser is especially advantageous for cutting pericardial tissue because the tissue absorbs and is vaporized by the $CO_2$ laser light but very little or no thermal energy is conducted to regions of the tissue that are not being cut. Only the boundary/edge of the cut is melted, effectively forming a weld.

In the preferred embodiment, a sheet of explanted equine pericardium is placed on the support surface 80. An operator directs the computer 74 to actuate the laser system 72, which cuts leaflets out of the sheet according to the prescribed pattern 96. To help maintain the tissue in good condition, it preferably is kept moist when being cut.

When cutting equine pericardium, the laser preferably is operated at a power of about 7.5 watts (joules/second). The laser can cut at a linear speed of about 25.4mm per second (1 inch per second), a pulse rate of about 39 pulses per mm (1000 pulses per inch) and a laser spot diameter of about 0.07mm (0.003 inches).

A measurement of laser energy per pulse is computed by using the following equation (1): [laser energy per pulse (joules/pulse)] = [power (joules/second)] / {[cutting speed (mm/second)] x [pulse rate (pulses/mm)]}.

For the above embodiment, the laser energy per pulse is about:

$$\text{[7.5 joules/second] / \{[25.4mm/second (1 inch/second)] x [39 pulses/mm (1000 pulses/inch)]\} = 0.0075 joules/pulse.}$$

Other materials, such as bovine or other kinds of explanted pericardium tissues and laminar materials can also be advantageously laser cut with a $CO_2$ laser as discussed above. With the invention, the materials are laser cut and about 0.005-0.5 joules of laser energy are supplied per pulse, with a laser spot size of about 0.05 to 0.13mm (0.002 to 0.005 inches) in diameter. In embodiments, the laser has a cutting speed of about 25.4mm/second (1 inch/second), and a pulse rate of about 39 pulses per mm (1000 pulses per inch). For the Universal Laser Systems M-series laser discussed above, the following sample settings enable laser cutting within the above-discussed parameters: a 1.5 Lens, 20% power setting, 3.4% speed, 39 pulses per mm and 39 dots per mm (1000 dots per inch).

It is to be understood that if parameters such as the pulse rate and cutting speed are adjusted, corresponding adjustments to other parameters can be made so that the energy imparted to the material substantially stays within the desired parameters. In this manner, a generally uniform weld can be formed along a cut edge without discoloring the edge or imparting excessive heat to other portions of the segment.

It is also to be understood that other types of lasers, such as an erbium laser that generates a laser beam having a wavelength of about 2.7-3.0pm, can suitably be used to cut segments. Such alternative lasers can be operated at settings so that the cut edges are welded as discussed above.

Alternative techniques may be employed for laser cutting of segments for use in prosthetics, such as described in US Patent Publication No. 2002/0091441.

Various types of explanted tissue and man-made materials can be cut with a laser by using generally the same principles as discussed above. For example, other types of laminar tissue can be cut so that the cut edges are welded and have a generally uniform consistency with little or no discoloration. Similarly, for man-made materials such as woven or knitted polymers, the cut edges preferably are melted so that fraying of the woven filaments or yarns is minimized or avoided, but discoloration is also avoided.

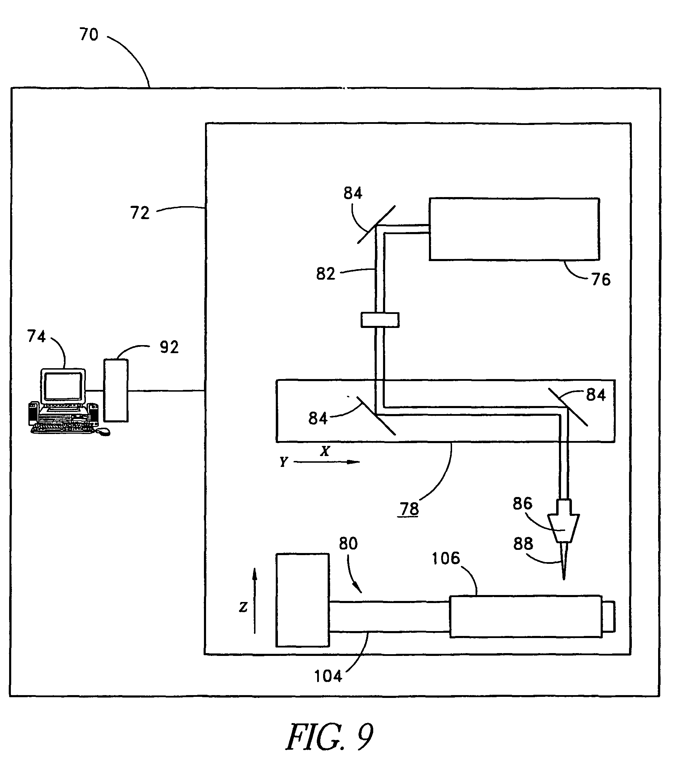

With reference next to Figure 9, an embodiment of a laser cutting apparatus for cutting curved or tubular materials is illustrated. This embodiment is substantially similar to the embodiment presented in Figure 6 except that the support surface 80 comprises a rotary axis 104 configured to accept a tubular source material 106. In addition to vertical movement about a Z-axis, the rotary axis 104 is adapted to rotate in order to help position the tubular source material 106 in an advantageous cutting position relative to the focused laser beam 88.

It will be appreciated that variations in, and modifications of, the embodiments described and illustrated may be made within the scope of the following claims.

**Claims**

1. A method of creating an implantable medical prosthesis, comprising:

   providing a sheet of flexible synthetic or explanted biological source material (90) having at least two tissue layers; and
   cutting a segment of tissue out of the sheet of source material with a laser beam (88);
   said cutting comprising operating a laser at a power and pulse rate such that said beam welds the layers of the source material together along a laser cut edge (100) without significantly burning the source material adjacent the cut edge:
   **characterised in that** the laser (72) is operated in a pulsed manner, supplying between about 0.005-0.5 joules of laser energy per pulse, with a laser spot size of about 0.05-0.13mm (0.002-0.005 inches) in diameter.

2. A method as claimed in Claim 1, wherein the laser beam power and pulse rate are selected so that there is substantially no discoloration of the source material along the cut edge (100).

3. A method as claimed in Claim 1 or Claim 2, comprising moving the laser beam (88) at a cutting speed of about 25.4mm per second (1 inch per second) and a pulse rate of about 39 pulses per mm (1000 pulses per inch).

4. A method as claimed in Claim 3, wherein the laser beam (88) supplies between about 0.005-0.02 joules of laser energy per pulse.

5. A method as claimed in Claim 4, wherein the laser beam operates at a wavelength of about 10.6 microns.

6. A method as claimed in Claim 4, wherein the laser beam operates at a wavelength of about 2.7-3.0 microns.

7. A method as claimed in Claim 4, wherein the laser energy is about 0.0075 joules per pulse and a laser spot diameter of about 0.07mm (0.003 inches).

8. A method as claimed in any preceding claim, wherein the source material (90) is explanted pericardium.

9. A method as claimed in Claim 8, wherein the pericardium comprises equine pericardium.

10. A method as claimed in any preceding claim, wherein the prosthesis comprises a heart valve, said cutting comprising cutting a plurality of segments of tissue out of the sheet of source material (90) with the laser beam (88) and attaching the cut segments to one another to form the valve.

11. Apparatus for creating an implantable medical prosthesis, said apparatus comprising:

    a laser system (72) for creating a focussed laser beam (88) for cutting a segment of tissue out of a sheet of a flexible source material (90) having at least two tissue layers;
    the laser system (72) operating a laser at a power and pulse rate such that the focussed laser beam (88) welds the layers of the source material together along a laser cut edge (100) without significantly burning the source material adjacent the cut edge;
    **characterised in that** the laser (72) is operated in a pulsed manner, supplying between about 0.005-0.5 joules of laser energy per pulse, with a laser spot size of about 0.05-0.13mm (0.002-0.005 inches) in diameter.

12. Apparatus as claimed in Claim 11, wherein the laser beam power and pulse rate are selected so that there is substantially no discoloration of the source material (90) along the cut edge (100).

13. Apparatus as claimed in Claim 11 or Claim 12, further comprising a motion system (78) arranged to move the focussed laser beam (88) at a cutting speed of about 25.4mm per second (1 inch per second), and wherein the laser is operated at a pulse rate of about 39 pulses per mm (1000 pulses per inch).

14. Apparatus as claimed in any of Claims 11 to 13, wherein the laser beam (88) supplies between about 0.005 to 0.02 joules of laser energy per pulse.

15. Apparatus as claimed in Claim 14, wherein the focussed laser beam (88) operates at a wavelength of about 10.6 microns.

16. Apparatus as claimed in Claim 14, wherein the focussed laser beam (88) operates at a wavelength of about 2.7-3.0 microns.

17. Apparatus as claimed in Claim 14, wherein the laser energy is about 0.0075 joules per pulse and the laser has a laser spot diameter of about 0.07mm (0.003 inches).

**Patentansprüche**

1. Verfahren zum Erzeugen einer implantierbaren, medizinischen Prothese, umfassend:

   Bereitstellen eines Bogens eines flexiblen, synthetischen oder explantierten biologischen Ausgangsmaterials (90) mit mindestens zwei Gewebeschichten; und
   Schneiden eines Gewebesegments aus dem Ausgangsmaterialbogen mit einem Laserstrahl (88);
   wobei das Schneiden den Betrieb eines Lasers bei einer solchen Leistung und Impulsrate umfasst, dass der Strahl die Schichten des Ausgangsmaterials entlang einer Laserschnittkante (100) zusammenschweißt, ohne das Ausgangsmaterial neben der Schnittkante wesentlich zu verbrennen:
   **dadurch gekennzeichnet, dass** der Laser (72) gepulst betrieben wird und eine Laserenergie von zwischen ca. 0,005 und 0,5 Joules pro Impuls mit einem Laserpunktgrößendurchmesser von ca. 0,05 bis 0,13mm (0,002-0,005 Inch) liefert.

2. Verfahren nach Anspruch 1, wobei die Laserstrahlleistung und die Impulsrate so gewählt sind, dass das Ausgangsmaterial entlang der Schnittkante (100) im Wesentlichen nicht verfärbt wird.

3. Verfahren nach Anspruch 1 oder 2, umfassend: Bewegen des Laserstrahls (88) bei einer Schneidgeschwindigkeit von ca. 25,4 mm pro Sekunde (1 Inch pro Sekunde) und einer Impulsrate von ca. 39 Impulsen pro mm (1000 Impulse pro Inch).

4. Verfahren nach Anspruch 3, wobei der Laserstrahl (88) eine Laserenergie von zwischen ca. 0,005 und 0,02 Joules pro Impuls liefert.

5. Verfahren nach Anspruch 4, wobei der Laserstrahl bei einer Wellenlänge von ca. 10,6 Mikrometern betrieben wird.

6. Verfahren nach Anspruch 4, wobei der Laserstrahl bei einer Wellenlänge von ca. 2,7 bis 3,0 Mikrometern betrieben wird.

7. Verfahren nach Anspruch 4, wobei die Laserenergie bei ca. 0,0075 Joules pro Impuls liegt und ein Laserpunktdurchmesser ca. 0,07 mm (0,003 Inch) misst.

8. Verfahren nach einem der vorhergehenden Ansprüche, wobei das Ausgangsmaterial (90) explantiertes Perikard ist.

9. Verfahren nach Anspruch 8, wobei das Perikard Pferdeperikard aufweist.

10. Verfahren nach einem der vorhergehenden Ansprüche, wobei die Prothese eine Herzklappe umfasst und das Schneiden das Schneiden einer Mehrzahl von Gewebesegmenten aus dem Bogen des Ausgangsmaterials (90) mit dem Laserstrahl (88) und Befestigen der geschnittenen Segmente miteinander zum Bilden der Klappe umfasst.

11. Vorrichtung zum Erzeugen einer implantierbaren medizinischen Prothese, wobei die Vorrichtung umfasst:

   ein Lasersystem (72) zum Erzeugen eines fokussierten Laserstrahls (88) zum Schneiden eines Gewebesegments aus einem Bogen eines flexiblen Ausgangsmaterials (90) mit mindestens zwei Gewebeschichten;
   dass das Lasersystem (72) einen Laser bei einer solchen Leistung und Impulsrate betreibt, dass der fokussierte Laserstrahl (88) die Schichten des Ausgangsmaterials entlang einer Laserschnittkante (100) zusammenschweißt, ohne das Ausgangsmaterial neben der Schnittkante wesentlich zu verbrennen;
**dadurch gekennzeichnet, dass** der Laser (72) gepulst betrieben wird und eine Laserenergie von zwischen ca. 0,005 und 0,5 Joules pro Impuls mit einem Laserpunktgrößendurchmesser von ca. 0,05 bis 0,13 mm (0,002 - 0,005 Inch) liefert.

12. Vorrichtung nach Anspruch 11, wobei die Laserstrahlleistung und die Impulsrate so gewählt werden, dass sich das Ausgangsmaterial (90) entlang der Schnittkante (100) im Wesentlichen nicht verfärbt.

13. Vorrichtung nach Anspruch 11 oder 12, die weiterhin ein Bewegungssystem (78) aufweist, das so angeordnet ist, dass es den fokussierten Laserstrahl (88) bei einer Schneidgeschwindigkeit von ca. 25,4 mm pro Sekunde (1 Inch pro Sekunde) bewegt und wobei der Laser bei einer Impulsrate von ca. 39 Impulsen pro mm betrieben wird (1000 Impulse pro Inch).

14. Vorrichtung nach einem der Ansprüche 11 bis 13, wobei der Laserstrahl (88) eine Laserenergie von zwischen ca. 0,005 und 0,02 Joules pro Impuls liefert.

15. Vorrichtung nach Anspruch 14, wobei der fokussierte Laserstrahl (88) bei einer Wellenlänge von ca. 10,6 Mikrometern betrieben wird.

16. Vorrichtung nach Anspruch 14, wobei der fokussierte Laserstrahl (88) bei einer Wellenlänge von ca. 2,7 bis 3,0 Mikrometern betrieben wird.

17. Vorrichtung nach Anspruch 14, wobei die Laserenergie ca. 0,0075 Joules pro Impuls beträgt und der Laser einen Laserpunktdurchmesser von ca. 0,07 mm (0,003 Inch) hat.

**Revendications**

1. Procédé de création d'une prothèse médicale implantable comprenant :

    la fourniture d'une feuille de matériau source (90) synthétique ou biologique explanté, flexible, ayant au moins deux couches de tissu ; et
    la découpe d'un segment de tissu dans la feuille de matériau source avec un faisceau laser (88) ;
    ladite découpe comprenant le fonctionnement d'un laser à une puissance et à un taux d'impulsion tels que ledit faisceau soude les couches du matériau source conjointement le long d'un bord de découpe au laser (100) sans brûler significativement le matériau source adjacent au bord de découpe ;
    **caractérisé en ce que** le laser (72) fonctionne de manière pulsée, fournissant entre environ 0,005 à 0,5 joules d'énergie laser par impulsion, avec une taille de point laser d'environ 0,05 à 0,13 mm (0,002 à 0,005 pouces) de diamètre.

2. Procédé selon la revendication 1, dans lequel la puissance du faisceau laser et le taux d'impulsion sont choisis de sorte qu'il ne se produise substantiellement aucune décoloration du matériau source le long du bord de découpe (100).

3. Procédé selon la revendication 1 ou 2, comprenant le déplacement du faisceau laser (88) à une vitesse de découpe d'environ 25,4 mm par seconde (1 pouce par seconde) et un taux d'impulsion d'environ 39 impulsions par mm (1000 impulsions par pouce).

4. Procédé selon la revendication 3, dans lequel le faisceau laser (88) fournit entre environ 0,005 à 0,02 joules d'énergie laser par impulsion.

5. Procédé selon la revendication 4, dans lequel le faisceau laser fonctionne à une longueur d'ondes d'environ 10,6 microns.

6. Procédé selon la revendication 4, dans lequel le faisceau laser fonctionne à une longueur d'ondes d'environ 2,7 à 3,0 microns.

7. Procédé selon la revendication 4, dans lequel l'énergie laser est d'environ 0,0075 joules par impulsion et un diamètre de point laser est d'environ 0,07 mm (0,003 pouces) .

8. Procédé selon l'une quelconque des revendications précédentes, dans lequel le matériau source (90) est un péricarde explanté.

9. Procédé selon la revendication 8, dans lequel le péricarde comprend un péricarde équin.

10. Procédé selon l'une quelconque des revendications précédentes, dans lequel la prothèse comprend une valvule cardiaque, ladite découpe comprenant la découpe de multiples segments de tissus dans la feuille de matériau source (90) avec le faisceau laser (88) et la fixation des segments découpés l'un à l'autre pour former la valvule.

11. Appareil de création d'une prothèse médicale implantable, ledit appareil comprenant :

un système laser (72) pour créer un faisceau laser focalisé (88) pour découper un segment de tissu dans une feuille d'un matériau source flexible (90) ayant au moins deux couches de tissu ;
le système laser (72) fonctionnant à une puissance et à un taux d'impulsion tels que le faisceau laser focalisé (88) soude les couches du matériau source conjointement le long d'un bord de découpe au laser (100) sans brûler significativement le matériau source adjacent au bord de découpe ;
**caractérisé en ce que** le laser (72) fonctionne de manière pulsée, fournissant entre environ 0,005 à 0,5 joules d'énergie laser par impulsion, avec une taille de point laser d'environ 0,05 à 0,13 mm (0,002 à 0,005 pouces) de diamètre.

12. Appareil selon la revendication 11, dans lequel la puissance de faisceau laser et le taux d'impulsion sont choisis de sorte qu'il ne se produise substantiellement aucune décoloration du matériau source (90) le long du bord de découpe (100).

13. Appareil selon la revendication 11 ou 12, comprenant en outre un système de déplacement (78) disposé pour déplacer le faisceau laser focalisé (88) à une vitesse de découpe d'environ 25,4 mm par seconde (1 pouce par seconde) et dans lequel le laser fonctionne à un taux d'impulsion d'environ 39 impulsions par mm (1000 impulsions par pouce).

14. Appareil selon l'une quelconque des revendications 11 à 13, dans lequel le faisceau laser (88) fournit entre environ 0,005 à 0,02 joules d'énergie laser par impulsion.

15. Appareil selon la revendication 14, dans lequel le faisceau laser focalisé (88) fonctionne à une longueur d'ondes d'environ 10,6 microns.

16. Appareil selon la revendication 14, dans lequel le faisceau laser focalisé (88) fonctionne à une longueur d'ondes d'environ 2,7 à 3,0 microns.

17. Appareil selon la revendication 14, dans lequel l'énergie laser est d'environ 0,0075 joules par impulsion et le laser a un diamètre de point laser d'environ 0,07 mm (0,003 pouces).

FIG. 1

FIG. 2

**FIG. 3**

**FIG. 4**

*FIG. 5*

EP 1 408 882 B1

FIG. 6

FIG. 7

FIG. 8

FIG. 9

**EP 1 408 882 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 6129758 A
- US 20020091441 A